Europäisches Patentamt

European Patent Office

Office européen des brevets

(11) Veröffentlichungsnummer: **0 028 309**
**B1**

(12) **EUROPÄISCHE PATENTSCHRIFT**

(45) Veröffentlichungstag der Patentschrift: **30.01.85**

(21) Anmeldenummer: **80105702.7**

(22) Anmeldetag: **23.09.80**

(51) Int. Cl.⁴: **C 12 P 33/10,** C 07 J 1/00, C 07 J 5/00, C 07 J 7/00, C 07 J 9/00

(54) **Verfahren zur Herstellung von 11-alpha-Hydroxy-20-alpha-hydroxymethyl-1,4-pregnadien-3-on.**

(30) Priorität: **02.10.79 DE 2940285**

(43) Veröffentlichungstag der Anmeldung:
**13.05.81 Patentblatt 81/19**

(45) Bekanntmachung des Hinweises auf die Patenterteilung:
**30.01.85 Patentblatt 85/05**

(84) Benannte Vertragsstaaten:
**AT BE CH DE FR GB IT LI LU NL SE**

(56) Entgegenhaltungen:
**CH-A- 383 364**
**DE-A-1 909 152**
**DE-B-1 128 854**
**GB-A- 843 218**
**US-A-3 352 760**

**W. CHARNEY und H.L. HERZOG: "Microbial transformations of steroids", 1967, Academic Press, New York und London, Seiten 298, 710, 717**

(73) Patentinhaber: **SCHERING AKTIENGESELLSCHAFT Berlin und Bergkamen Müllerstrasse 170/178 Postfach 65 03 11 D-1000 Berlin 65 (DE)**

(72) Erfinder: **Petzoldt, Karl, Dr.**
**Flachsweg 10**
**D-1000 Berlin 38 (DE)**
Erfinder: **Weber, Alfred, Dr.**
**Schützallee 56**
**D-1000 Berlin 37 (DE)**
Erfinder: **Kieslich, Klaus, Dr.**
**Fischhausenweg 4**
**D-3300 Braunschweig (DE)**
Erfinder: **Krieger, Bernhard, Dr.**
**Martinstrasse 20**
**D-4750 Unna (DE)**

(56) References cited:
**CHEMICAL ABSTRACTS,Eighth collective Index, Subjects Columbus, Ohio, US, Band 66-75, 1967-1971, Seiten 25278s, Spalte 3 "Phosphonic - Propana" 11alpha, 17,21-trihydroxy, formation of, by Aspergillus ochraceus**

Courier Press, Leamington Spa, England.

# 0 028 309

## Beschreibung

Die Erfindung betrifft das im Patentanspruch gekennzeichnete Verfahren.

Es ist bekannt, daß man die 11$\alpha$-Hydroxylierung im allgemeinen bei Steroiden durchführt, die in der 1,2-Position gesättigt sind. Fermentiert man 20$\alpha$-Hydroxymethyl-4-pregnen-3-on mit einer Kultur von Aspergillus occhraceus so erhält man als Hauptprodukt das 6$\alpha$,11$\alpha$-Dihydroxy-20-hydroxymethyl 4-pregnen-3-on, während das gewünschte 11$\alpha$-Hydroxy-20-hydroxymethyl-4-pregnen-3-on in nur sehr geringen Ausbeuten erhalten wird.

Es wurde nun gefunden, das eine 6$\beta$-Hydroxylierung praktisch nicht auftritt, wenn man anstelle des in der 1,2-Position gesättigten Steroids das entsprechende in der 1-Stellung ungesättigte Steroid als Substrat verwendet.

Die für die Umsetzung benötigten Aspergillus occhraceus Stämme stehen der Fachwelt bei anerkannten Mikroorganismen-Sammlungen frei zur Verfügung. Geeignete Aspergillus occhraceus Stämme sind beispielsweise: Aspergillus occhraceus CBS 13 252, ATCC 1008, ATCC 12 337, ATCC 18 500 oder NRRL 405.

Das erfindungsgemäße Verfahren wird unter den Bedingungen durchgeführt, die man üblicherweise bei der mikrobiologischen Hydroxylierung von Steroiden anwendet.

Unter den üblicherweise verwendeten Kulturbedingungen werden in einem geeigneten Nährmedium unter Belüften, Submerskulturen angezüchtet. Dann setzt man der Kulturen das Substrat (in einem Lösungsmittel gelöst oder in emulgierter Form) zu und fermentiert, bis eine maximale Substratumwandlung erreicht ist.

Geeignete Substratlösungsmittel sind beispielsweise, Methanol, Äthanol, Glykolmonomethyläther, Dimethylformamid, oder Dimethylsulfoxyd. Die Emulgierung des Substrates kann beispielsweise bewirkt werden, indem man diese in mikronisierter Form in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispeil Äthylenoxyaddukte oder Fettsäureester von Polyglykolen. Als geeignete Emulgatoren seien die handelsüblichen Netzmittel Tegin®, Tween®, und Span beispielsmäßig genannt.

Die optimale Substratkonzentration, Substratzugabezeit und Fermentationsdauer ist von den angewendeten Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Das nachfolgende Ausführungsbeispiel dient zur Erläuterung des erfindungsgemäßen Verfahrens.

### Beispiel 1

Ein 2 l Erlenmeyerkolben, der 1 Liter einer sterilisierten Nährlösung aus 3,0 % Glucose, 1,0 % Corn steep, 0,2 % Natriumnitrat, 0,1 % Kaliumdihydrogenphosphat, 0,2 % Dikaliumhydrogenphosphat, 0,05 % Magnesiumphosphat, 0,002 % Eisenphosphat und 0,05 % Kaliumchlorid, wird mit einer Lyophilkultur von Aspergillus occhraceus (NRRL 405) beimpft und 72 Stunden bei 30°C auf einem Rotationsschüttler geschüttelt. Mit 125 ml dieser Vorkultur wird dann ein 20 l Fermenter beimpft, der 7,5 l eines sterilisierten Nährmediums enthält, das aus 1 % Corn steep und 1 % Sojabohnenmehl besteht. Unter Zugabe von Silicon SH als Antischaummittel wird bei 29°C unter Belüftung (10 Liter pro Minute), 0,7 atü Druck und Rühren (220 Umdrehungen pro Minute) 24 Stunden germiniert. Mit 1,8 Liter dieser Vorfermenter-Kultur wird anschließend unter sterilen Bedingungen ein 30 l Fermenter mit 26 l sterilem Nährmedium, bestehen aus 1,0 % Corn steep und 1,25 % Sojabohnenmehl, beimpft und nach Zugabe von Antischaummittel bei 29°C unter Belüftung (10 Liter pro Minute), 0,7 atü Druck und Rühren (220 Umdrehungen pro Minute) 12 Stunden anwachsen lassen. Danach gibt man eine sterilisierte Suspension von 90 g feinstgemahlenem 20$\alpha$-Hydroxymethyl-1,4-pregnadien-3-on in 2 l Wasser hinzu und rührt und belüftet weiter. Nach 60 Stunden Kontaktzeit wird der Fermenterinhalt dreimal mit je 10 l Methylisobutylketon ausgerührt und die vereinigten Extrakte bei 50°C Badtemperatur im Vakuum konzentriert. Dabei fällt die entstandene 11$\alpha$-Hydroxy-Verbindung in Form eines weißen Kristallisats aus. Nach dem Absaugen und Trocknen erhält man 81 g 11$\alpha$-Hydroxy-20$\alpha$-hydroxymethyl-1,4-pregnadien-3-on vom Schmelzpunkt 251—252°C.

## Patentanspruch

Verfahren zur Herstellung von 11$\alpha$-Hydroxy-20$\alpha$-hydroxymethyl-1,4-pregnadien-3-on, dadurch gekennzeichnet, daß man 20$\alpha$-Hydroxymethyl-1,4-pregnadien-3-on mit einer lebenden Kultur von Aspergillus occhraceus, die zu der entsprechenden Umsetzung fähig ist, fermentiert.

## Revendication

Procédé de préparation de l'hydroxy-11$\alpha$ hydroxyméthyl-20$\alpha$ prégnadiène-1,4 one-3 caractérisé en ce qu'on fait fermenter l'hydroxyméthyl-20$\alpha$ prégnadiène-1,4 one-3 avec une culture vivante

2

d'Aspergillus occhraceus apte à effectuer la réaction correspondante.

**Claim**

Process for manufacturing 11α-hydroxy-20α-hydroxymethyl-1,4-pregnadien-3-one, characterised in that 20α-hydroxymethyl-1,4-pregnadien-3-one is fermented with a live culture of *Aspergillus occhraceus* which is capable of the appropriate reaction.